(19) Europäisches Patentamt
European Patent Office
Office européen des brevets

(11) Numéro de publication: 0 193 445 B1

(12) **FASCICULE DE BREVET EUROPEEN**

(45) Date de publication du fascicule du brevet: 09.05.90

(51) Int. Cl.[5]: C07K 5/00, A61K 37/64

(21) Numéro de dépôt: 86400272.0

(22) Date de dépôt: 10.02.86

(54) **Dérivés peptidiques, analogues de la pepstatine, inhibiteurs de la rénine et des protéases acides.**

(30) Priorité: 12.02.85 FR 8501982
12.02.85 FR 8501981

(43) Date de publication de la demande:
03.09.86 Bulletin 86/36

(45) Mention de la délivrance du brevet:
09.05.90 Bulletin 90/19

(84) Etats contractants désignés:
AT BE CH DE FR GB IT LI LU NL SE

(56) Documents cités:
EP-A- 0 114 993
FR-A- 2 531 951

(73) Titulaire: SANOFI, 40, Avenue George V, F-75008 Paris(FR)
Titulaire: INSTITUT NATIONAL DE LA SANTE ET DE LA RECHERCHE MEDICALE (INSERM), 101, rue de Tolbiac, F-75654 Paris Cédex 13(FR)

(72) Inventeur: Wagnon, Jean Le Hameau de la Rauze-Villa 34, Avenue du Pont Trinquat, F-34000 Montpellier(FR)
Inventeur: Callet, Georges, Cité Fleurie-Bâti. 2 Avenue de Maurin, F-34000 Montpellier(FR)
Inventeur: Gagnol, Jean Pierre, Place de l'Eglise, F-34000 Montpellier(FR)
Inventeur: Nisato, Dino, 4, Impasse de l'Olivette, F-34680 St.Georges d'Orques(FR)
Inventeur: Cazaubon, Catherine, 9, rue Pierre Boissier, F-34000 Montpellier(FR)

(74) Mandataire: Gillard, Marie-Louise et al, Cabinet Beau de Loménie 55, Rue d'Amsterdam, F-75008 Paris(FR)

EP 0 193 445 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne des nouveaux dérivés peptidiques inhibiteurs de la rénine et des protéases acides. Elle concerne également un procédé pour leur obtention et leur application en thérapeutique.

En 1970, UMEZAWA a isolé, à partir d'une culture de streptomyces, un pentapeptide désigné sous le nom de Pepstatine dont la structure a été établie ultérieurement et répond à la formule:

Isovaléryl – L-valyl – L-valyl – Statyl – L-valyl – Statine dans laquelle on désigne sous le nom de "statine" un acide aminé non usuel, l'acide (3S, 4S) amino-4 hydroxy-3 méthyl-6 heptanoïque.

Il a été montré que la pepstatine est un inhibiteur de protéases acides et agit en particulier sur la pepsine, la cathepsine D et la rénine. Spécialement, la rénine, enzyme d'origine rénale, intervient dans la séquence angiotensinogène, angiotensine I, angiotensine II au niveau de la transformation de l'angiotensinogène en angiotensine.

L'angiotensine II étant un puissant agent vasoconstricteur, intervient dans la régulation de la pression artérielle. On a envisagé d'utiliser la pepstatine pour lutter contre l'hypertension artérielle chez l'homme. Toutefois, la pepstatine agissant sur l'ensemble des protéases acides et présentant une faible solubilité en milieux aqueux et une faible affinité pour la rénine, son emploi en thérapeutique s'est avéré difficile. Des dérivés de la pepstatine ont été décrits dans la littérature scientifique. Par exemple, on a tenté de solubiliser la pepstatine par allongement de la chaîne peptidique (J. Cardiovasc. Pharmacol, 1980, 2, 687–698).

Dans le brevet Fr. 2 531 951 on a décrit des dérivés de la pepstatine présentant un niveau d'activité élevé sur l'inhibition de la rénine et de la pepsine. Ces dérivés répondent à la formule générale:

$$R–X–Y\text{-Statyl-Ala-Statyl-}OR' \quad (I)$$

dans laquelle X et Y, identiques ou différents, désignent des acides aminés de configuration L ou D, choisis parmi la valine, la leucine, l'isoleucine, la phénylalanine, la tyrosine, le tryptophane, l'histidine et la proline avec la condition que X et Y ne peuvent pas désigner simultanément la valine, R est H ou un groupe acylant ou N-protecteur fixé sur le radical aminoterminal de l'amino-acide X, tel que par exemple un groupe t-butoxycarbonyl (BOC), benzyloxycarbonyle (Z) ou adamantyloxycarbonyle (Adoc). Ces groupes assurent une protection labile en milieu acide.

On a maintenant trouvé des nouveaux dérivés peptidiques dont le modèle est également construit sur celui de la pepstatine. Dans certains cas, l'introduction d'acides aminés non naturels permet d'augmenter la solubilité de ces composés et leur résistance à la protéolyse. De manière tout à fait surprenante, on a trouvé que les peptides selon l'invention qui portent des résidus hydrophiles sur différents sites présentent un niveau d'activité élevé et très supérieur à celui de la pepstatine en tant qu'inhibiteur de la rénine humaine.

La présente invention a pour objet des peptides présentant un niveau élevé d'activité en tant qu'inhibiteurs de la rénine et d'autres protéases acides.

Dans la présente description et dans les revendications, les abréviations suivantes seront utilisées:

Acides aminés et groupes protecteurs ou activateurs.

Ces abréviations sont en accord avec celles indiquées par le Commission de Nomenclature de l'IUPAC-IUB section Biochimie. Les recommandations les plus récentes sont rapportées dans Eur. J. Biochem., 1984, 138, 5-7 et 9-37.

<u>Acides aminés et dérivés:</u>

Ala : Alanine
Asn : Asparagine
Asp : Acide Aspartique
Gln : Glutamine
Gly : Glycine
His : Histidine
Ile : Isoleucine
Leu : Leucine
Met : Methionine
Nle : Norleucine
Nva : Norvaline
Phe : Phénylalanine
Phg : Phénylglycine
Ser : Sérine
Sta : Statine
AHPPA : Acide amino-4 hydroxy-3 phényl-5 pentanoïque

ACHPA : Acide amino-4 cyclohexyl-5 hydroxy-3 pentanoïque
Met($O_2$) : méthionine dioxyde
Abu : Acide amino-2 butyrique

Ces acides aminés sont, sauf indication contraire, de configuration L.
Sta, AHPPA, ACHPA sont, sauf indication contraire, de configuration 3S, 4S.

Groupes protecteurs et activateurs :
Ac : Acétyle
Boc : t.butyloxycarbonyle
$(Boc)_2O$ : Anhydride bis-terbutyloxycarbonique
HONSu : N-hydroxysuccinimide
OEt : Ester éthylique
OMe : Ester méthylique
ONp : Ester p-nitrophénylique
ONSu : Ester de N-hydroxysuccinimide
OTcp : Ester trichloro-2,4,5 phénylique
iVa : Isovaléryle
Z : Benzoyloxycarbonyle
De plus, on utilisera les abréviations suivantes :
AcOET : Acétate d'éthyle
AcOH : Acide acétique
Bop : Hexafluorophosphate de benzyloxy tris diméthylaminophosphonium
CCM : Chromatographie en couche mince
DCCI : Dicyclohexylcarbodiimide
DCHA : Dicyclohexylamine
DCU : Dicyclohexylurée
DIPEA : Diisopropyléthylamine
DMF : Diméthylformamide
DMSO : Diméthylsulfoxyde
Ether : Ether éthylique
HOBt : Hydroxy-1 benzotriazole
$KHSO_4$-$K_2SO_4$ : Solution aqueuse contenant 16,6 g de bisulfate de potassium et 33,3 g de sulfate de potassium pour 1 litre
MeOH : Méthanol
NEM : N-éthyl morpholine
NMM : N-méthyl morpholine
TA : Température ambiante
TFA : Acide trifluoracétique
min : minutes
h : heures

Les composés selon l'invention répondent à la formule générale suivante :

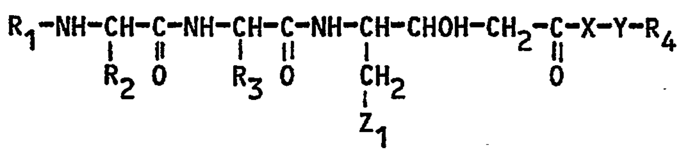

(I)

dans laquelle:
$R_1$ représente l'un des groupes suivants:

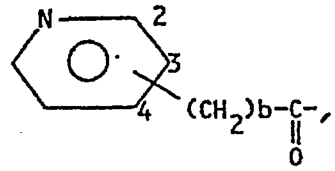 où b = 0, 1, 2, 3, 4, 5 ou 6

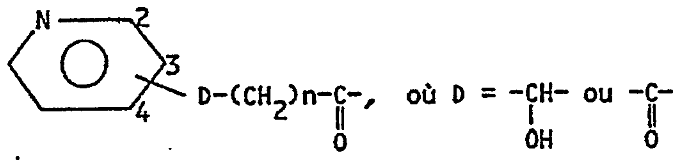

où D = … ou …

et n = 1, 2, 3, 4 ou 5

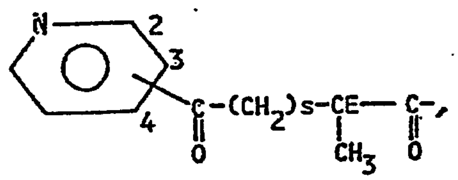 où E = $-H$ ou $-CH_3$

et s = 1, 2, 3 ou 4

$R_2$ représente un groupe alkyle inférieur non substitué ou substitué par un phényle, naphtyle, cyclohexyle, ou pyridyle ou $R_2$ représente un radical phényle, naphtyle, cyclohexyle ou pyridyle;

$R_3$ représente un alkyle inférieur non substitué ou substitué par un groupe amino libre ou portant un groupe protecteur, par un carboxyle libre, par un groupe hydroxy, par un groupe alkylthio inférieur, par un phényle, par un naphtyle ou par un imidazolyl-4;

$R_4$ représente un hydroxyle, un alcoxy inférieur, un benzyloxy ou un groupe amino libre ou substitué par 1 ou 2 alkyles inférieurs;

$Z_1$ représente l'isopropyle, le phényle ou le cyclohexyle formant respectivement avec le radical

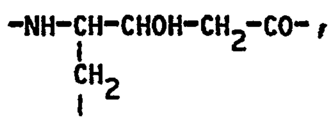

le résidu de l'aminoacide statine, à savoir l'acide (3S, 4S) amino-4 hydroxy-3 méthyl-6 heptanoïque ou de l'acide (3S, 4S) amino-4 hydroxy-3 phényl-5 pentanoïque (AHPPA) ou de l'acide (3S, 4S) amino-4 cyclohexyl-5 hydroxy-3 pentanoïque (ACHPA);

X–Y est un dipeptide choisi parmi Ala–Sta, Ala–Leu, Leu–Phe, Val–Sta, Abu–Sta, Ile–Ser, Phg–Sta.

La présente invention comprend également les sels éventuels pharmaceutiquement acceptables des peptides de formule (I) avec des acides minéraux ou organiques ou des métaux alcalins ou alcalino-terreux.

La terme "alkyle" désigne les radicaux d'hydrocarbures aliphatiques saturés ou insaturés contenant 1 à 10 atomes de carbone. Les groupes "alkyle" préférés aux fins de l'invention sont les groupes alkyle inférieur tels que définis ci-après.

Les expressions "alkyle inférieur", "alcényle inférieur" et "alkylidène inférieur", telles qu'utilisées ici, désignent les radicaux d'hydrocarbures aliphatiques saturés ou insaturés contenant jusqu'à 6 atomes de carbone.

Les expressions "alcoxy inférieur" et "alkylthio inférieur" représentent les groupes hydroxyle et thiol substitués par un groupe alkyle inférieur tel que défini ci-dessus.

L'expression "hétérocycle monocyclique à 5 ou 6 chaînons" inclut la pyrrolidine, l'imidazole, le thiazole, le thiophène, le furanne, le pyrrole, le triazole, l'oxazole, l'isoxazole, la pyridine, les thiadiazoles.

Par "groupe protecteur" on entend un groupe protecteur utilisé normalement dans la chimie des peptides, par exemple Boc, Z ou iVa.

L'expression "groupe acyle" utilisée pour la définition de $R_1$ inclut les résidus d'acides carboxyliques hétérocycliques. Des groupes acyles préférés sont le résidu d'un acide carboxylique dont le carboxyle est lié à un hétérocycle monocyclique à 5 ou 6 chaînons, notamment les groupes pipéridinylcarboxyliques, les groupes picolinoyle, nicotinoyle et isonicotinoyle et le résidu d'acides alcanoïques ou alcénoïques, tels que l'acide acétique, l'acide propionique, l'acide butyrique, l'acide valérique et leurs dérivés oméga-hydroxy ou oméga-oxo, substitués en position oméga par un hétérocycle monocyclique à 5 ou 6 chaînons comme exemplifiés ci-dessus.

Particulièrement préférés sont les dérivés peptidiques de formule (I) dans laquelle $R_2$, $R_3$, $R_4$, X, Y et $Z_1$ sont tels que définis ci-dessus et $R_1$ représente un groupe acyle choisi parmi:

- (pyridyl-2)-4 oxo-4 butyryle,
- (pyridyl-2)-4 hydroxy-4-butyryle,
- (pyridyl-3)-3 propinyle,
- (pyridyl-3)-4 butyryle,
- nicotinoyle,
- benzène sulfonyle.

Les produits selon l'invention peuvent être préparés selon les méthodes habituelles de la chimie des peptides. Plus particulièrement, à partir d'un composé de formule:

$$H-Y-R'_4$$

dans laquelle $R'_4$ représente un alcoxy inférieur, un benzyloxy ou un groupe amino libre ou substitué par un ou 2 alkyles inférieurs et Y est choisi parmi les résidus des acides aminés Sta, Leu, Phe et Ser, on couple, étape par étape, les divers acides aminés convenablement protégés, le produit obtenu à chaque étape étant déprotégé, selon des procédés connus, avant d'être soumis à un nouveau couplage, chacune des opérations de couplage étant effectuée en utilisant soit un ester activé de l'aminoacide à coupler, soit l'aminoacide N-protégé en présence de dicyclohexyl-carbodiimide. Le produit de départ est avantageusement un ester d'alkyle inférieur de l'acide aminé C terminal sur lequel est condensé l'aminoacide suivant de la séquence. Après libération de la fonction amine du dipeptide, on procède à l'élongation de la chaîne peptidique par couplage de l'acide aminé suivant, convenablement protégé. Chaque phase de couplage est suivie d'une opération sélective de libération de l'amine qui va entrer en réaction lors de la création de la liaison peptidique suivante. Les différentes opérations de couplage sont effectuées soit en utilisant un ester activé de l'aminoacide à coupler, soit en utilisant l'aminoacide N-protégé en présence de dicyclohexyl carbodiimide. Les phases de déprotection sélective de l'amine sont effectuées selon la nature du groupe protecteur utilisé soit par hydrogénolyse, soit par acidolyse en milieu acide fort tel que l'acide trifluoracétique. Lorsque l'acide aminé à introduire dans la séquence possède dans sa chaîne latérale une fonction susceptible de réagir, il convient de bloquer celle-ci par un groupe protecteur convenable que l'on élimine ultérieurement.

La protection de l'aminoacide initial par le groupe $R_1$ s'effectue selon des méthodes connues, avant le couplage du résidu :

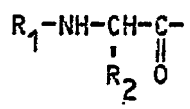

avec l'aminoacide suivant.

Les peptides (I) sous forme acide ($R_4$ = OH) peuvent être obtenus à partir des esters correspondants par saponification en milieu alcalin dilué. On peut également préparer leurs sels. Les peptides (I) sous forme amide ($R_4 = NH_2$ ou $R_4 = N(Alk)_2$) sont obtenus directement en prenant comme produit de départ les aminoacides sous forme amide qui existent commercialement.

Selon le schéma général, il est possible soit de préparer l'ensemble de la séquence désirée, soit de préparer 2 fragments de cette séquence que l'on couple finalement pour obtenir le peptide désiré.

Les sels éventuels pharmaceutiquement acceptables des peptides selon l'invention avec des acides minéraux ou organiques ou des métaux alcalins ou alcalino-terreux sont formés par des méthodes classiques.

Les composés de la présente invention ont une action inhibitrice sur l'activité rénine plasmatique humaine très importante et de façon générale nettement supérieure à celle du produit naturel : la pepstatine; à ce titre ils peuvent être utilisés dans le traitement de l'hypertension artérielle.

Ils possèdent également une action inhibitrice marquée sur les protéases acides, notamment la pepsine. On peut donc envisager l'utilisation des produits selon l'invention dans les domaines thérapeutiques où l'inhibition de tels systèmes enzymatiques est justifiée, notamment, outre l'hypertension artérielle, l'ulcère gastroduodénal et les affections inflammatoires.

La présente invention a aussi pour objet les compositions pharmaceutiques antihypertenseurs contenant les peptides de formule (I) ou leurs sels pharmaceutiquement acceptables en tant que principe actif.

Les peptides de la présente invention peuvent être utilisés en thérapeutique par voie injectable : intraveineuse, intramusculaire ou sous-cutanée. Ils sont utilisés dans un solvant tel que le sérum physiologique (solution saline isotonique) ou dans un tampon tel que le tampon phosphate ; ils peuvent également être mis en suspension dans un agent diluant, aqueux ou non aqueux, pharmaceutiquement acceptable. Chaque unité de dosage peut contenir de 1 à 1000 mg de principe actif.

La quantité de principe actif à utiliser varie suivant les effets thérapeutiques recherchés, la gravité de l'affection à traiter et la voie d'administration choisie. Elle doit être déterminée pour chaque patient et est le plus souvent comprise entre 0,100 g et 2 g de principe actif.

Les exemples suivants, non limitatifs, sont donnés à titre d'illustration de la présente invention.

Dans tous ces exemples, le pH des solutions dans un solvant organique est mesuré ou contrôlé en utilisant du papier pH indicateur humide.

Les points de fusion indiqués (Fc) sont mesurés au tube capillaire.

Enfin, les spectres de résonance magnétique nucléaire ont été enregistrés à 250 MHz en solution dans le DMSO, l'étalon interne étant l'hexaméthyldisiloxane.

Les abréviations suivantes sont utilisées :

s : singulet
d : doublet
m : multiplet ou massif
q : quadruplet.
De plus, . H ar signifie H aromatique
. H im signifie H imidazolique
. H pyr signifie H de la pyridine.

Les déplacements chimiques (delta) sont mesurés en ppm.

Exemple 1

N-(Nicotinoyl)-Phe-Nle-Sta-Ala-Leu-OMe.
SR 42873.

1. Boc-Ala-Leu-OMe.

Dans 50 ml de $CH_2Cl_2$, on dissout successivement à TA 2,86 g de Boc-Ala-ONSu, 1,81 g de Leu-OMe, HCl et 1,15 g de NEM. On vérifie que le pH est à 6-7, sinon on l'ajuste par adjonction de NEM. On agite 4 h à TA, puis lave successivement la solution organique par $KHSO_4$-$K_2SO_4$ à 5 %, de l'eau, $NaHCO_3$ à 5 %, on sèche sur $Na_2SO_4$ puis on évapore le solvant et on obtient une huile.

Rendement : 2,93 g (92 %).

2. Ala-Leu-OMe, TFA.

360 mg du produit précédent sont recouverts de 5 ml de TFA. Après 20 min, le TFA est évaporé, on reprend le résidu par un mélange, à volume égal, pentane-éther. Par grattage, un solide blanc apparaît, il est essoré, rincé à l'éther, séché.

Rendement : 320 mg (85 %).

3. Boc-Sta-Ala-Leu-OMe.

330 mg du sel de TFA précédemment obtenu sont solubilisés dans 30 Ml de dioxanne contenant 230 mg de NEM, on ajoute 275 mg de Boc-Sta-OH, 206 mg de DCCI et 135 mg de HOBt, le pH est ajusté à une valeur de 6-7 par NEM si nécessaire, puis on agite 24 h à TA. On filtre la DCU, évapore le solvant, dissout le résidu dans un mélange, à volume égal, AcOEt-hexane et chromatographie sur une colonne de gel de silice 60 Merck dans le même mélange de solvant en éluant par 250 ml du même mélange de solvant, puis 250 ml du mélange dans la proportion 75/25 en volume, puis 100 ml d'AcOEt. Les fractions contenant le produit sont évaporées, reprises dans l'éther, essorées, séchées.

Rendement : 380 mg (80 %).

4. Boc-Phe-Nle-Sta-Ala-Leu-OMe.

Ce composé est préparé en utilisant les mêmes méthodes de couplage que précédemment.

5. SR 42873

Le produit obtenu à l'étape précédente (200 mg) est placé dans 3 ml de TFA à 0°C pendant 10 min. Le sel de TFA obtenu après le traitement habituel est dissous dans 15 ml de $CH_2Cl_2$, le pH est amené à 7 par addition de NMM, on ajoute 34 mg d'acide nicotinique, 56 mg de DCCI et 42 mg d'HOBt. Après 24 h, on traite de façon habituelle. Le résidu est chromatographié sur silice par élution avec AcOEt. On obtient une fraction homogène de 45 mg.

SPECTRE DE RMN

| Delta | Aspect | Protons | Attribution |
|---|---|---|---|
| 7 et 8,95 | m | 14 H | 5 NH, 4 H pyr<br>5 H ar |
| 4,86 | d, J=4 Hz | 1 H | 1 H, OH (Sta) |
| 4,25 | m | 3 H | H, alpha (Phe, Nle, Leu) |
| 3,8 | s large | 2 H | 2 H alpha (Sta, Ala) |
| 3,55 | s | 3 H | $OCH_3$ |

Exemple 2

N-((pyridyl-2)-4 oxo-4 butyryl)-Phe-Nle-Sta-Leu-Phe-OMe (SR 42793).

1. N-((pyridyl-2)-4 oxo-4 butyryl)-Phe-OMe

Dans 10 ml d'acétonitrile, on agite 1 g de Phe-OMe, HCl, 830 mg d'acide (pyridyl-2) oxo-4 butyrique en présence de 2,05 g de Bop et de 940 mg de NEM. Après 3 jours, on évapore à siccité, ajoute de l'eau et AcOEt, lave plusieurs fois par de l'eau carbonatée, de l'eau, de l'eau saline, sèche et évapore. On isole 1,25 g de solide qui est purifié par chromatographie sur gel de silice en éluant par un mélange à volume égal de pentane et AcOEt. Après recristallisation dans un mélange $CH_2Cl_2$-$Et_2O$, on obtient 870 mg du produit attendu.

2. N-((pyridyl-2)-4 oxo-4 butytyl)-Phe-OH.

810 mg de l'ester précédent dans 15 ml de méthanol sont hydrolysés pendant 4 h à TA par 190 mg de soude dans 5 ml d'eau. On évapore à siccité, ajoute de l'eau carbonatée, extrait par AcOEt puis acidifie à pH 4,65 en contrôlant au pH-mètre. On extrait ensuite par AcOEt, lave à l'eau, l'eau saline et évapore. Le solide blanc obtenu est recristallisé du mélange MeOH-$Et_2O$ ; Fc = 115-120°C.

3. SR 42793.

On opère ensuite selon les méthodes précédemment décrites pour obtenir le produit attendu.

SPECTRE DE RMN

| Delta | Aspect | Protons | Attribution |
|---|---|---|---|
| 8,67-7,05 | m | 19 H | 5 NH CO<br>10 H ar<br>$C_6H_5$, 4 H ar<br>$C_5H_4N$ |
| 4,78 | d, J=4 Hz | 1 H | OH (Sta) |
| 4,50-4,11 | m | 4 H | CH (alpha) |
| 3,81-3,67 | m | 2 H | C<u>H</u>OH (Sta)<br>C<u>H</u>NH (Sta) |
| 3,48 | s | 3 H | $OCH_3$ |
| 2,13-2,00 | m | 2 H | $CH_2CO$ (Sta) |
| 1,71-1,05 | m | 12 H | CH, $CH_2$ aliphatiques |
| 0,89-0,68 | m | 15 H | $CH_3$ (NLe)<br>$(CH_3)_2CH$ (Leu)<br>$(CH_3)_2CH$ (Sta) |

Exemple 3

(R,S) N-((Pyridyl-2)-4 hydroxy-4 butyryl)Phe-Nle-Sta-Leu-Phe-OMe (SR 42991).

Ce composé est obtenu par réduction du SR 42793. On dissout 23 mg de SR 42793 dans 2 ml de MeOH et on ajoute 10 mg de $BH_4Na$ à TA. Après 18 h, on ajoute une solution de $KHSO_4$ en excès, agite pendant 10 min puis ajoute de l'eau carbonatée et évapore MeOH à froid. On extrait par 3 volumes d'AcOEt, lave à l'eau et sèche sur $K_2SO_4$. A l'évaporation, on isole un solide qui est trituré dans le mélange éther-hexane. On obtient 16 mg du produit attendu.

SPECTRE DE RMN

| Delta | Aspect | Protons | Attribution |
|---|---|---|---|
| 8,43-7,0 | m | 19 H | 5 NH CO<br>10 H ar<br>$C_6H_5$, 4 H ar<br>$C_5H_4N$ |
| 5,34 | d, J=4 Hz | 1 H | OH |
| 4,80 | d, J=4 Hz | 1 H | OH (Sta) |
| 4,70-4,00 | m | 5 H | CH (alpha) |
| 3,80-3,66 | m | 2 H | C<u>H</u>OH (Sta) |
| 3,48 | s | 3 H | $OCH_3$ |
| 2,13-0,63 | m | 33 H | |

En utilisant les mêmes méthodes de couplage, on a préparé les composés suivants :

| N° SR | Formule |
|---|---|
| 43108 | N-((pyridyl-3)-4 oxo-4 butyryl)-Phe-Nle-Sta-Leu-Phe-OMe |
| 43123 | (R,S) N-((pyridyl-3)-4 hydroxy-4 butyryl)-Phe-Nle-Sta-Leu-Phe-OMe |
| 43282 | N-((pyridyl-3) acétyl)-Phe-Nle-Sta-Leu-Phe-OMe |
| 43314 | N-((pyridyl-2) acétyl)-Phe-Nle-Sta-Leu-Phe-OMe |
| 43454 | N-((pyridyl-3)-3 propionyl)-Phe-Nle-Sta-Leu-Phe-OMe |
| 43484 | N-((pyridyl-2)-4 butyryl)-Phe-Nle-Sta-Leu-Phe-OMe |
| 43519 | N-((N-Boc pipéridinyl-3) acétyl)-Phe-Nle-Sta-Leu-Phe-OMe |
| 43633 | N-((pyridyl-4)-3 propionyl)-Phe-Nle-Sta-Leu-Phe-OMe |
| 43781 | N-((pyridyl-4) acétyl)-Phe-Nle-Sta-Leu-Phe-OMe |
| 43782 | N-((pyridyl-3)-4 butyryl)-Phe-Nle-Sta-Leu-Phe-OMe |

Ces produits sont caractérisés par leur spectre de RMN.

SPECTRE DE RMN DU SR 43108

| Delta | Aspect | Protons | Attribution |
|---|---|---|---|
| 9,02-7 | m | 19 H | 10 H $C_6H_5$, 4 H $C_5H_4N$ 5 H NHCO |
| 4,80 | d, J=4 Hz | 1 H | OH (Sta) |
| 4,52-4,09 | m | 4 H | CH (alpha) |
| 3,85-3,70 | m | 2 H | C<u>H</u>OH et C<u>H</u>NH (Sta) |
| 3,48 | s | 3 H | $OCH_3$ |
| 2,12-2,00 | m | 2 H | $CH_2CO$ (Sta) |
| 1,70-1,08 | m | 12 H | CH et $CH_2$ aliphatiques (Nle, Leu, Sta) |
| 0,92-0,71 | m | 15 H | $CH_3$ (Nle, Leu, Sta) |

SPECTRE DE RMN DU SR 43123

| Delta | Aspect | Protons | Attribution |
|---|---|---|---|
| 8,43-7,00 | m | 19 H | 10 H $C_6H_5$ 4 H $C_5H_4N$ 5 H NHCO |
| 5,33 | d, J=4 Hz | 1 H | OH |
| 4,83 | d, J=4 Hz | 1 H | OH (Sta) |
| 4,72-3,70 | m | 7 H | CH (alpha) C<u>H</u>OH (Sta) C<u>H</u>NH (Sta) |
| 3,50 | s | 3 H | $OCH_3$ |
| 2,15-0,70 | m | 33 H | |

SPECTRE DE RMN DU SR 43282

| Delta | Aspect | Protons | Attribution |
|---|---|---|---|
| 8,41-7,02 | m | 19 H | $C_6H_5$, $C_5H_4N$ NHCO |
| 4,85 | d, J=4 Hz | 1 H | OH |
| 4,61-4,12 | m | 4 H | CH alpha |
| 3,84-3,68 | m | 2 H | CHNH et CHOH (Sta) |
| 3,49 | s | 3 H | $OCH_3$ |
| 3,45-3,18 | m | | $CH_2C_5H_4N$ (+$H_2O$ de DMSO) |
| 3,02-2,61 | m | 4 H | $CH_2C_6H_5$ |
| 2,13-2,0 | m | 2 H | $CH_2CO$ (Sta) |
| 1,68-1,04 | m | 12 H | $CH_2$ (Nle) ; CH et $CH_2$ (Sta et Leu) |
| 0,85-0,66 | m | 15 H | $CH_3$ (Nle, Sta, Leu) |

SPECTRE DE RMN DU SR 43314

| Delta | Aspect | Protons | Attribution |
|---|---|---|---|
| 8,42-7,02 | m | 19 H | $C_6H_5$, $C_5H_4N$, NHCO |
| 4,82 | d, J=4 Hz | 1 H | OH |
| 4,60-4,14 | m | 4 H | CH alpha |
| 3,85-3,70 | m | 2 H | CHNH et CHOH (Sta) |
| 3,53 | s | 2 H | $CH_2C_5H_4N$ |
| 3,49 | s | 3 H | $OCH_3$ |
| 3,04-2,63 | m | 4 H | $CH_2C_6H_5$ |
| 2,11-2,00 | m | 2 H | $CH_2CO$ (Sta) |
| 1,72-1,08 | m | 12 H | $CH_2$ (Nle) ; CH et $CH_2$ (Sta et Leu) |
| 0,82-0,64 | m | 15 H | $CH_3$ (Nle, Sta, Leu) |

SPECTRE DE RMN DU 43454

| Delta | Aspect | Protons | Attribution |
|---|---|---|---|
| 8,36-7,075 | m | 19 H | $C_5H_4N$, $C_6H_5$ NHCO |
| 4,84 | d, J=4 Hz | 1 H | OH |
| 4,57-4,12 | m | 4 H | CH alpha (Phe, Leu, Nle) |
| 3,84-3,67 | m | 2 H | C<u>H</u>NH, C<u>H</u>OH (Sta) |
| 3,49 | s | 3 H | $OCH_3$ |
| 3,03-2,57 | m | 6 H | $CH_2C_6H_5$, $CH_2C_5H_4N$ |
| 2,31 | t, J env. 6 Hz | 2 H | $-CH_2-CH_2-CO$ |
| 2,13-2,03 | m | 2 H | $CH_2CO$ (Sta) |
| 1,70-1,09 | m | 12 H | $CH_2$ (Nle), CH, $CH_2$ (Leu), CH, $CH_2$ (Sta) |
| 0,85-0,66 | m | 15 H | $CH_3$ |

SPECTRE DE RMN DU 43484

| Delta | Aspect | Protons | Attribution |
|---|---|---|---|
| 8,54-7,04 | m | 19 H | H ar, NHCO |
| 4,87 | d, J=4 Hz | 1 H | OH |
| 4,61-4,14 | m | 4 H | CH alpha (Phe,Nle-Leu) |
| 3,87-3,70 | m | 2 H | CHNH, CHOH (Sta) |
| 3,50 | s | 3 H | $OCH_3$ |
| 3,04-2,62 | m | 4 H | $\underline{CH_2}C_6H_5$ |
| 2,62-2,41 | m | | $\underline{CH_2}C_5H_4N$ (+DMSO) |
| 2,14-1,95 | m | 4 H | $CH_2CO$ (Sta) |
| 1,78-1,10 | m | 14 H | $C_5H_4N-CH_2CH_2-\underline{CH_2}$ $C_5H_4N-CH_2-\underline{CH_2}-\overline{CH_2}$, $CH_2$ (Nle) CH, $CH_2$ (Sta, Leu) |
| 0,91-0,67 | m | 15 H | $CH_3$ |

SPECTRE DE RMN DU SR 43633

| Délta | Aspect | Protons | Attribution |
|---|---|---|---|
| 8,37-8,15 | m | 3 H | 2 H ortho $C_5H_4N$, NHCO |
| 8,18-8,03 | m | 2 H | NHCO |
| 7,75 | d, J=8 Hz | 1 H | NHCO |
| 7,37 | d, J=8 Hz | 1 H | NHCO (Sta) |
| 7,27-7,00 | m | 10 H<br>2 H | $C_6H_5$<br>$C_5H_4N$ |
| 4,84 | d, J=4 Hz | 1 H | OH (Sta) |
| 4,59-4,13 | m | 4 H | CH alpha (Phe, Nle, Leu) |
| 3,87-3,68 | m | 2 H | C<u>H</u>NH et C<u>H</u>OH (Sta) |
| 3,50 | s | 3 H | $OCH_3$ |
| 3,00-2,58 | m | 6 H | $CH_2C_6H_5$<br>$C\underline{H_2}C_5H_4N$ |
| 2,38-2,00 | m | 4 H | $CH_2$-$C\underline{H_2}$-CO<br>$CH_2CO$ (Sta) |
| 1,71-0,61 | m | 27 H | CH, $CH_2$, $CH_3$ (Nle, Leu, Sta) |

SPECTRE DE RMN DU SR 43782

| Delta | Aspect | Protons | Attribution |
|---|---|---|---|
| 8,37-8,23 | m | 3 H | 2 H ortho<br>$C_5H_4N$, NHCO |
| 8,10-7,97 | 2 d, J=8 Hz | 2 H | NHCO |
| 7,73 | d, J=8 Hz | 1 H | NHCO |
| 7,50-7,05 | m | 10 H | $C_6H_5$ |
| | | 2 H | $C_5H_4N$ |
| | | 1 H | NHCO |
| 4,80 | d, J=4 Hz | 1 H | OH (Sta) |
| 4,60-4,15 | m | 4 H | CH alpha (Phe,<br>Nle, Leu) |
| 3,80-3,70 | m | 2 H | $C\underline{H}NH$ et<br>$C\underline{H}OH$ (Sta) |
| 3,49 | s | 3 H | $OCH_3$ |
| 3,05-2,6 | m | 4 H | $C\underline{H_2}C_6H_5$ |
| 2,40-1,92 | m | 6 H | $C_5H_4NC\underline{H_2}$<br>$C\underline{H_2}$-CO (Sta) |
| 1,70-0,6 | m | 29 H | $-CH_2-CH_2-CH_2CO$<br>$-CH_2CH_2CH_2-CO$<br>et CH, $CH_2$, $CH_3$<br>(Leu, Nle, Sta) |

Les produits selon l'invention ont été étudiés en ce qui concerne leurs propriétés thérapeutiques et notamment leur action inhibitrice enzymatique. Plus particulièrement, les composés ont été évalués "in vitro" sur l'inhibition de l'Activité Rénine Plasmatique Humaine (A.R.P.).

I - Méthode

La méthode d'évaluation est inspirée de GUYENE (J. Clin. Endocrinol. Metab., 1976, 43, 1301) dans le mesure où l'inhibition de l'A.R.P. est évaluée à partir d'un pool de plasmas humains, riche en rénine (15 à 20 ml d'angiotensine I libérée par millilitre et par heure) incubée pendant 60 min à 37°C, dans un tampon phosphate à pH 7,4, en présence de concentrations croissantes du produit à étudier.

Le plasma humain contient le substrat :
l'angiotensinogène et l'enzyme : la rénine. L'angiotensine I libérée en cours de réaction est mesurée par dosage radio-immunologique à l'aide d'un kit : Plasma Renin Activity Kit de Travenol (n° CA 533553). Un inhibiteur de l'enzyme de conversion, le fluorure de phénylméthylsulfonyle(PMSF), est ajouté au milieu d'incubation. Le volume total d'incubation est de 555 microlitres répartis ainsi :

- 420 microlitres de plasma humain
- 11 à 50 microlitres du produit à étudier à des concentrations variables

- 119 à 80 microlitres de tampon phosphate
- 5 microlitres de PMSF.

On prépare une solution d'acide acétique dans le méthanol (19/1 en volume) et une solution de soude dans le méthanol (2/1 en volume). Dans un mélange équivolumique de ces 2 solutions, on prépare une solution mère du peptide à 0,001M. Les dilutions ultérieures du peptide sont alors effectuées dans le tampon phosphate.

La quantité de solvant présente dans une solution du peptide à une concentration inférieure à 0,0001M n'interfère pas avec les résultats.

II-Résultats

Les résultats sont exprimés par la dose de composé évaluée en moles, qui inhibe de 50 % ($IC_{50}$) l'Activité Rénine Plasmatique Humaine observée en l'absence d'inhibiteur.

Les résultats obtenus avec divers produits de l'invention sont représentés dans le tableau (I) suivant où figurent les $IC_{50}$ de chaque molécule en ce qui concerne leur inhibition de l'Activité Rénine Plasmatique Humaine à pH 7,4. De 5 á 10 doses ont été nécessaires pour déterminer ces $IC_{50}$. La pepstatine, en tant que substance de référence, est toujours testée en parallèle dans chaque expérience. Les résultats sont exprimés par leur valeur logarithmique (-Log $IC_{50}$).

Tableau I

| N° SR | Inhibition A.R.P. Humaine -Log $IC_{50}M$ pH 7,4 |
|---|---|
| Pepstatine | 4,92 |
| 42 793 | 6,05 |
| 42 873 | 7,09 |
| 42 991 | 7,23 |
| 43 108 | 6,35 |
| 43 123 | 7,09 |
| 43 282 | 7,10 |
| 43 314 | 6,53 |
| 43 454 | 7,17 |
| 43 384 | 7,00 |
| 43 519 | 6,76 |
| 43 633 | 6,69 |
| 43 781 | 6,88 |
| 43 782 | 7,38 |

La toxicité des produits selon l'invention est compatible avec leur utilisation en thérapeutique.

**Revendications pour les états contractants: BE, CH, DE, FR, GB, IT, LI, LU, NL, SE**

1. Un dérivé peptidique de formule:

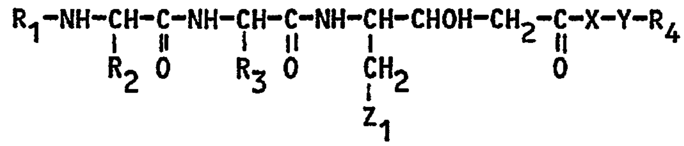

(I)

dans laquelle:

$R_1$ représente l'un des groupes suivants:

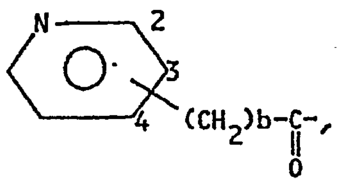

où b = 0, 1, 2, 3, 4, 5 ou 6

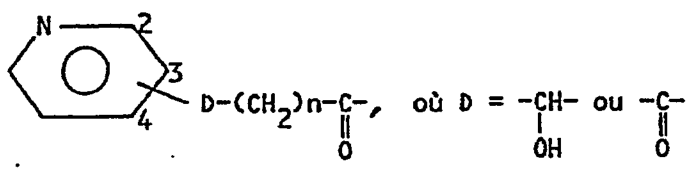

et n = 1, 2, 3, 4 ou 5

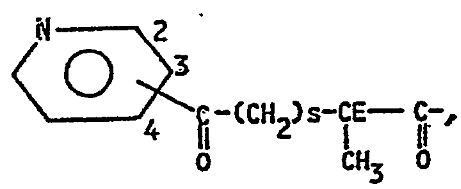

où E = –H ou –$CH_3$ et s = 1, 2, 3 ou 4

$R_2$ représente un groupe alkyle contenant jusqu'à 6 atomes de carbone non substitué ou substitué par un phényle, naphtyle, cyclohexyle, ou pyridyle ou $R_2$ représente un radical phényle, naphtyle, cyclohexyle ou pyridyle;

$R_3$ représente un alkyle contenant jusqu'à 6 atomes de carbone non substitué ou substitué par un groupe amino libre ou portant un groupe protecteur, par un carboxyle libre, par un groupe hydroxy, par un groupe alkylthio contenant jusqu'à 6 atomes de carbone, par un phényle, par un naphtyle ou par un imidazolyl-4;

$R_4$ représente un hydroxyle, un alcoxy contenant jusqu'à 6 atomes de carbone, un benzyloxy ou un groupe amino libre ou substitué par 1 ou 2 alkyles contenant jusqu'à 6 atomes de carbone;

$Z_1$ représente l'isopropyle, le phényle ou le cyclohexyle formant respectivement avec le radical

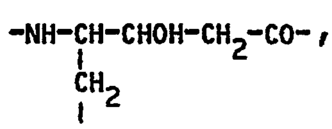

le résidu de l'aminoacide statine, à savoir l'acide (3S, 4S) amino-4 hydroxy-3 méthyl-6 heptanoïque ou de l'acide (3S, 4S) amino-4 hydroxy-3 phényl-5 pentanoïque (AHPPA) ou de l'acide (3S, 4S) amino-4 cyclohexyl-5 hydroxy-3 pentanoïque (ACHPA);

X–Y est un dipeptide choisi parmi Ala–Sta, Ala–Leu, Leu–Phe, Val–Sta, Abu–Sta, Ile–Ser, Phg–Sta;

ou un des sels éventuels pharmaceutiquement acceptables dudit dérivé peptidique avec les acides minéraux ou organiques ou avec des métaux alcalins ou alcalino-terreux.

2. Dérivé peptidique selon la revendication 1, caractérisé en ce que $R_1$ est choisi parmi les groupements suivants:

– (pyridyl-2)-4 oxo-4 butyryle,

– (pyridyl-2)-4 hydroxy-4 butyryle,

– (pyridyl-3)-3 propionyle,

– (pyridyl-3)-4 butyryle,

– nicotinoyle.

3. Dérivé peptidique caractérisé en ce qu'il est le N-((N-Boc-pipéridinyl-3)-acétyl-Phe-Nle-Sta-Leu-Phe-OMe.

4. Procédé de préparation d'un dérivé peptidique selon la revendication 1, caractérisé en ce qu'à partir d'un composé de formule: H–Y–R'$_4$ dans laquelle R'$_4$ représente un alcoxy contenant jusqu'à 6 atomes de carbone, un benzyloxy ou un groupe amino libre ou substitué par 1 ou 2 alkyles contenant jusqu'à 6 atomes de carbone et Y est choisi parmi les résidus des acides aminés Sta, Leu, Phe et Ser, on couple, étape par étape, les divers acides aminés ou des fragments de la séquence dudit peptide convenablement protégés, le produit obtenu à chaque étape étant déprotégé, selon les procédés connus, avant d'être soumis à un nouveau couplage, chacune des opérations de couplage étant effectuée en utilisant soit un ester activé de l'aminoacide à coupler, soit l'aminoacide N-protégé en présence de dicyclohexylcarbodiimide, en ce qu'éventuellement on saponifie le composé obtenu pour obtenir le peptide de formule I dans laquelle $R_4$ = OH et/ou en ce qu'éventuellement on transforme le peptide obtenu en l'un de ses sels pharmaceutiques acceptables avec des acides minéraux ou organiques ou des métaux alcalins ou alcalino-terreux.

5. Procédé pour l'obtention du dérivé peptidique de formule N-((N-Boc-piperidinyl-3)acétyl)-Phe-Nle-Sta-Leu-Phe-OMe, caractérisé en ce qu'à partir d'un composé de formule H–Y–R'$_4$ dans laquelle R'$_4$ représente un méthoxy, X est Phe, on couple étape par étape, les divers acides aminés ou des fragments de la séquence dudit peptide convenablement protégés, le produit obtenu à chaque étape étant déprotégé, selon les procédés connus, avant d'être soumis à un nouveau couplage, chacune des opérations de couplage étant effectuée en utilisant, soit un ester activé de l'aminoacide à coupler, soit l'aminoacide N-protégé en présence de dicyclohexylcarbodiimide.

6. Compositions pharmaceutiques, caractérisées en ce qu'elles contiennent un produit selon la revendication 1 en tant qu'ingrédient actif.

7. Compositions pharmaceutiques selon la revendication 6, utilisables notamment pour le traitement de la tension artérielle, caractérisées en ce qu'elles contiennent de 1 à 1000 mg de produit selon la revendication 1 par unité de dosage en mélange avec un excipient pharmaceutique.

**Revendications pour l'état contractant: AT**

1. Procédé pour l'obtention de dérivés peptidiques de formule:

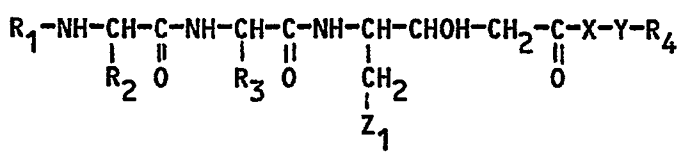

(I)

dans laquelle:

$R_1$ représente l'un des groupes suivants:

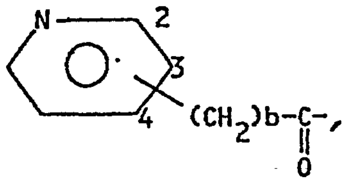

où b = 0, 1, 2, 3, 4, 5 ou 6

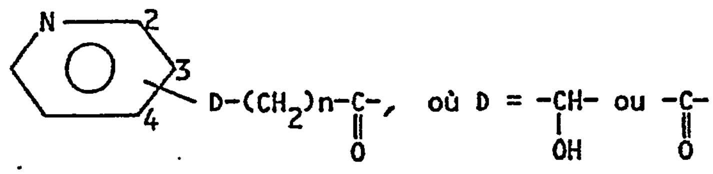

et n = 1, 2, 3, 4 ou 5

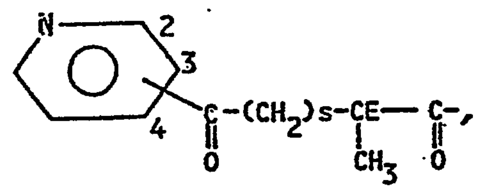

où E = -H ou $-CH_3$ et s = 1, 2, 3 ou 4

$R_2$ représente un groupe alkyle contenant jusqu'à 6 atomes de carbone non substitué ou substitué par un phényle, naphtyle, cyclohexyle, ou pyridyle ou $R_2$ représente un radical phényle, naphtyle, cyclohexyle ou pyridyle;

$R_3$ représente un alkyle contenant jusqu'à 6 atomes de carbone non substitué ou substitué par un groupe amino libre ou portant un groupe protecteur, par un carboxyle libre, par un groupe hydroxy, par un groupe alkylthio contenant jusqu'à 6 atomes de carbone, par un phényle, par un naphtyle ou par un imidazolyl-4;

$R_4$ représente un hydroxyle, un alcoxy contenant jusqu'à 6 atomes de carbone, un benzyloxy ou un groupe amino libre ou substitué par 1 ou 2 alkyles contenant jusqu'à 6 atomes de carbone;

$Z_1$ représente l'isopropyle, le phényle ou le cyclohexyle formant respectivement avec le radical

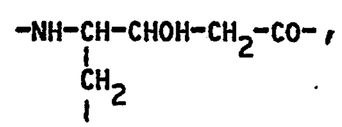

le résidu de l'aminoacide statine, à savoir l'acide (3S, 4S) amino-4 hydroxy-3 méthyl-6 heptanoïque ou de l'acide (3S, 4S) amino-4 hydroxy-3 phényl-5 pentanoïque (AHPPA) ou de l'acide (3S, 4S) amino-4 cyclohexyl-5 hydroxy-3 pentanoïque (ACHPA);

X–Y est un dipeptide choisi parmi Ala–Sta, Ala–Leu, Leu–Phe, Val–Sta, Abu–Sta, Ile–Ser, Phg–Sta; ou un des sels éventuels pharmaceutiquement acceptables dudit dérivé peptidique avec les acides minéraux ou organiques ou avec des métaux alcalins ou alcalino-terreux, caractérisé en ce qu'à partir d'un composé de formule: H–Y–R'$_4$ dans laquelle R'$_4$ représente un alcoxy contenant jusqu'à 6 atomes de carbone, un benzyloxy ou un groupe amino libre ou substitué par 1 ou 2 alkyles contenant jusqu'à 6 atomes de carbone et Y est choisi parmi les résidus des acides aminés Sta, Leu, Phe et Ser, on couple, étape par étape, les divers acides aminés ou des fragments de la séquence dudit peptide convenablement protégés, le produit obtenu à chaque étape étant déprotégé, selon les procédés connus, avant d'être soumis à un nouveau couplage, chacune des opérations de couplage étant effectuée en utilisant, soit un ester activé de l'aminoacide à coupler, soit l'aminoacide N-protégé en présence de dicyclohexylcarbodiimide, en ce qu'éventuellement on saponifie le composé obtenu pour former le peptide de formule I dans laquelle $R_4$ = OH et/ou en ce qu'éventuellement on transforme le peptide obtenu en l'un de ses sels pharmaceutiquement acceptables avec des acides minéraux ou organiques ou des métaux alcalins ou alcalino-terreux.

2. Procédé selon la revendication 1, caractérisé en ce que le dernier acide aminé à coupler est choisi de sorte que $R_1$ représente l'un des groupes suivants:
– (pyridyl-2)-4 oxo-4 butyryle,
– (pyridyl-2)-4 hydroxy-4 butyryle,
– (pyridyl-3)-3 propionyle,

– (pyridyl-3)-4 butyryle,
– nicotinoyle.

3. Procédé pour l'obtention du dérivé peptidique de formule N-((N-Boc-piperidinyl-3)-acétyl)-Phe-Nle-Sta-Leu-Phe-OMe caractérisé en ce qu'à partir d'un composé de formule H–Y–R'$_4$ dans laquelle R'$_4$ représente un méthoxy, Y est Phe, on couple étape par étape, les divers acides aminés ou des fragments de la séquence dudit peptide convenablement protégés, le produit obtenu à chaque étape étant déprotégé, selon les procédés connus, avant d'être soumis à un nouveau couplage, chacune des opérations de couplage étant effectuée en utilisant, soit un ester activé de l'aminoacide à coupler, soit l'aminoacide N-protégé en présence de dicyclohexylcarbodiimide.

4. Utilisation des peptides préparés par le procédé selon l'une quelconque des revendications 1 à 3 pour la fabrication de compositions pharmaceutiques pour le traitement de la tension artérielle.

**Claims for the contracting states: BE, CH, DE, FR, GB, IT, LI, LU, NL, SE**

1. A peptide derivative of the formula:

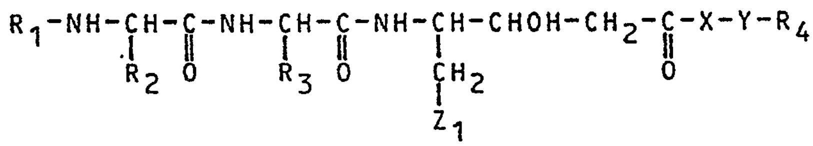
(I)

in which:

$R_1$ represents one of the following groups:

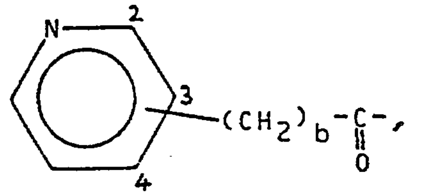
in which b = 0,1,2,3,4,5 or 6

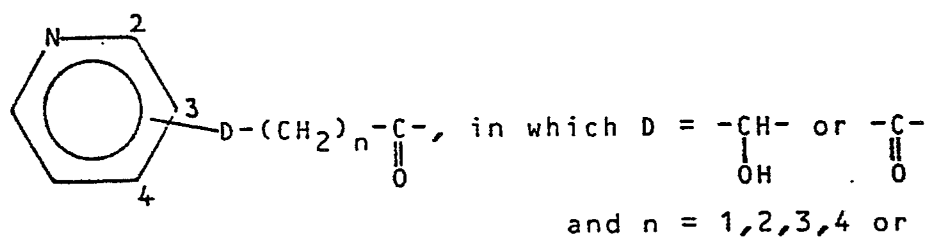
in which D =
or
and n = 1,2,3,4 or 5

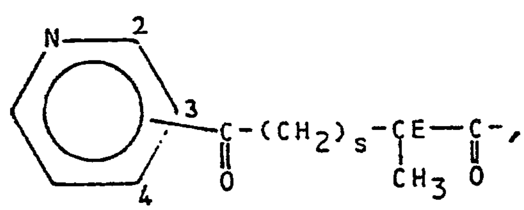
in which E = $-H$ or $-CH_3$
and s = 1,2,3 or 4

$R_2$ represents an alkyl group containing up to 6 carbon atoms which is unsubstituted or substituted by a phenyl, naphthyl, cyclohexyl or pyridyl, or $R_2$ represents a phenyl, naphthyl, cyclohexyl or pyridyl radical;

$R_3$ represents an alkyl containing up to 6 carbon atoms which is unsubstituted or substituted by a free amino group or an amino group carrying a protecting group, by a free carboxyl, by a hydroxyl group, by a lower alkylthio group containing up to 6 carbon atoms, by a phenyl, by a naphthyl or by an imidazol-4-yl;

$R_4$ represents a hydroxyl, an alkoxy containing up to 6 carbon atoms, a benzyloxy or a free amino group or an amino group substituted by one or 2 alkyls containing up to 6 carbon atoms;

$Z_1$ represents isopropyl, phenyl or cyclohexyl, respectively forming with the radical:

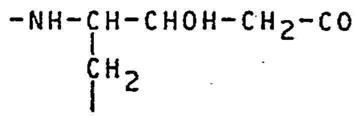

the residue of the amino acid statin, namely (3S,4S)-4-amino-3-hydroxy-6-methylheptanoic acid or of (3S,4S)-4-amino-3-hydroxy-5-phenylpentanoic acid (AHPPA) or of (3S,4S)-4-amino-5-cyclohexyl-3-hydroxypentanoic acid (ACHPA);

X–Y is a dipeptide chosen from the group comprising Ala–Sta, Ala–Leu, Leu–Phe, Val–Sta, Abu–Sta, Ile–Ser and Phg–Sta;

or one of any pharmaceutically acceptable salts of the said peptide derivative with mineral or organic acids or with alkali metals or alkaline earth metals.

2. A peptide derivative according to claim 1, characterized in that $R_1$ is chosen from the following groups:

– 4-(pyridin-2-yl)-4-oxobutyryl,
– 4-(pyridin-2-yl)-4-hydroxybutyryl,
– 3-(pyridin-3-yl)propionyl,
– 4-(pyridin-3-yl)butyryl,
– nicotinoyl.

3. A peptide derivative, characterized in that it is the N-((-N-Boc-piperidin-3-yl)acetyl)-Phe-Nle-Sta-Leu-Phe-OMe.

4. A process for the preparation of a peptide derivative according to claim 1, characterized in that, starting from a compound of the formula:

$$H–Y–R'_4$$

in which $R'_4$ represents an alkoxy containing up to 6 carbon atoms, a benzyloxy or free amino group or an amino group substituted by one or two alkyls containing up to 6 carbon atoms, and Y is chosen from the residues of the amino acids, Sta, Leu, Phe and Ser, the various amino acids or fragments of the sequence of the said peptide, appropriately protected, are coupled in a stepwise fashion, the product obtained at each step being deprotected according to known processes before being subjected to a new coupling, and each of the coupling operations being carried out using either an activated ester of the amino acid to be coupled or the N-protected amino acid in the presence of dicyclohexylcarbodiimide, in that, if appropriate, the compound obtained is saponified to form the peptide of the formula (I) in which $R_4$ = OH, and/or in that, if appropriate, the peptide obtained is converted to one of its pharmaceutically acceptable salts with mineral or organic acids or alkali metals or alkaline earth metals.

5. A process for the preparation of a peptide derivative of formula: N-((N-Boc-piperidin-3-yl)acetyl)-Phe-Nle-Sta-Leu-Phe-OMe, characterized in that starting from a compound of the formula $H–Y–R'_4$ in which $R'_4$ represents a methoxy, Y is Phe, the various amino acids or fragments of the sequence of the said peptide, appropriately protected, are coupled in a stepwise fashion, the product obtained at each step being deprotected according to known processes before being subjected to a new coupling, and each of the coupling operations being carried out using either an activated ester of the amino acid to be coupled or the N-protected amino acid in the presence of dicyclohexylcarbodiimide.

6. Pharmaceutical compositions, characterized in that they contain, as the active ingredient, a product according to claim 1.

7. Pharmaceutical compositions according to claim 6, which can be used especially for the treatment of arterial tension, characterized in that they contain from 1 to 1000 mg of a product according to claim 1, per dosage unit, mixed with a pharmaceutical excipient.

**Claims for the contracting state: AT**

1. A process for the preparation of peptide derivatives of the formula:

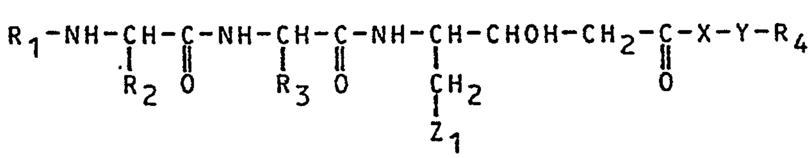
(I)

in which:

$R_1$ represents one of the following groups:

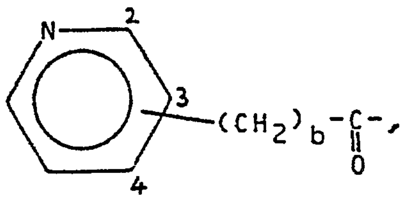, in which b = 0,1,2,3,4,5 or 6

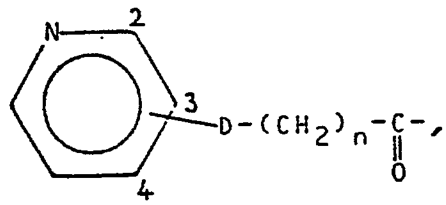, in which D = 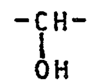 or 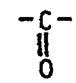

and n = 1,2,3,4 or 5

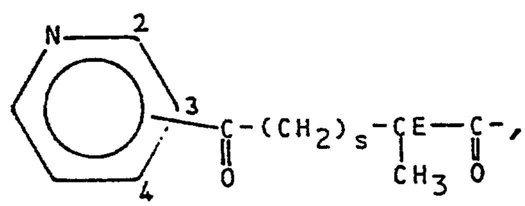, in which E = $-H$ or $-CH_3$

and s = 1,2,3 or 4

$R_2$ represents an alkyl group containing up to 6 carbon atoms which is unsubstituted or substituted by a phenyl, naphthyl, cyclohexyl or pyridyl, or $R_2$ represents a phenyl, naphthyl, cyclohexyl or pyridyl radical;

$R_3$ represents an alkyl containing up to 6 carbon atoms which is unsubstituted or substituted by a free amino group or an amino group carrying a protecting group, by a free carboxyl, by a hydroxyl group, by a lower alkylthio group containing up to 6 carbon atoms, by a phenyl, by a naphthyl or by an imidazol-4-yl;

$R_4$ represents a hydroxyl, an alkoxy containing up to 6 carbon atoms, a benzyloxy or a free amino group or an amino group substituted by one or 2 alkyls containing up to 6 carbon atoms;

$Z_1$ represents isopropyl, phenyl or cyclohexyl, respectively forming with the radical:

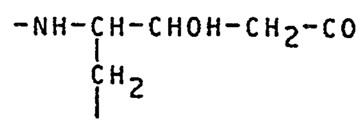

the residue of the amino acid statin, namely (3S,4S)-4-amino-3-hydroxy-6-methylheptanoic acid or of (3S,4S)-4-amino-3-hydroxy-5-phenylpentanoic acid (AHPPA) or of (3S,4S)-4-amino-5-cyclohexyl-3-hydroxypentanoic icid (ACHPA);

X–Y is a dipeptide chosen from the group comprising Ala–Sta, Ala–Leu, Leu–Phe, Val–Sta, Abu–Sta, Ile–Ser and Phg–Sta;

or one of any pharmaceutically acceptable salts of the said peptide derivative with mineral or organic acids or with alkali metals or alkaline earth metals, characterized in that, starting from a compound of the formula:

$$H\text{–}Y\text{–}R'_4$$

in which $R'_4$ represents an alkoxy containing up to 6 carbon atoms, a benzyloxy or a free amino group or an amino group substituted by one or two alkyls containing up to 6 carbon atoms, and Y is chosen from the residues of the amino acids Sta, Leu, Phe and Ser, the various amino acids or fragments of the sequence of the said peptide, appropriately protected, are coupled in a stepwise fashion, the product obtained at each step being deprotected according to known processes before being subjected to a new coupling, and each of the coupling operations being carried out using either an activated ester of the amino acid to be coupled or the N-protected amino acid in the presence of dicyclohexylcarbodiimide, in that, if appropriate, the compound obtained is saponified to form the peptide of the formula (I) in which $R_4$ = OH, and/or in that, if appropriate, the peptide obtained is converted to one of its pharmaceutically acceptable salts with mineral or organic acids or alkali metals or alkaline earth metals.

2. A process according to claim 1, characterized in that the last amino acid to be coupled is chosen in a manner that $R_1$ represents one of the following groups:
– 4-(pyridin-2-yl)-4-oxobutyryl,
– 4-(pyridin-2-yl)-4-hydroxybutyryl,
– 3-(pyridin-3-yl)propionyl,
– 4-(pyridin-3-yl)butyryl,
– nicotinoyl.

3. A process for the preparation of a peptide derivative of formula: N-((-N-Boc-piperidin-3-yl)acetyl)-Phe-Nle-Sta-Leu-Phe-OMe, characterized in that starting from a compound of the formula H–Y–R'$_4$ in which R'$_4$ represents a methoxy, Y is Phe, the various amino acids or fragments of the sequence of the said peptide, appropriately protected, are coupled in a stepwise fashion, the product obtained at each step being deprotected according to known processes before being subjected to a new coupling, and each of the coupling operations being carried out using either an activated ester of the amino acid to be coupled or the N-protected amino acid in the presence of dicyclohexylcarbodiimide.

4. Use of the peptides prepared by the process according to any one of claims 1 to 3 for the preparation of pharmaceutical compositions for the treatment of arterial tension.

**Patentansprüche für die Vertragsstaaten: BE, CH, DE, FR, GB, IT, LI, LU, NL, SE**

1. Peptidderivat der Formel

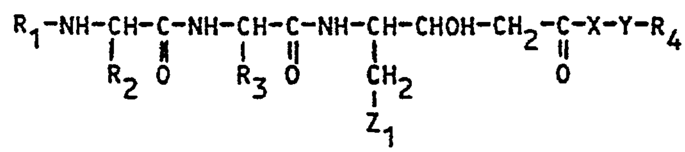

(I)

worin:

$R_1$ eine der folgenden Gruppen darstellt:

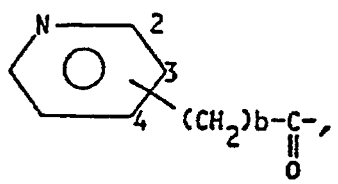

worin b = 0, 1, 2, 3, 4, 5 oder 6

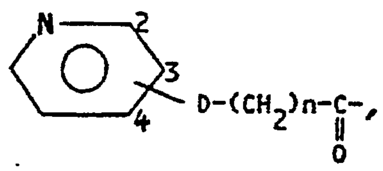

worin D =

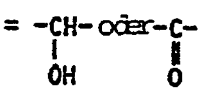

und n = 1, 2, 3, 4 oder 5

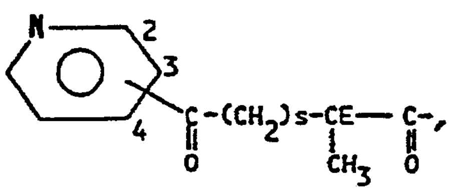

worin E = -H oder $-CH_3$
und s = 1, 2, 3 oder 4

$R_2$ eine nicht substituierte oder durch Phenyl, Naphthyl, Cyclohexyl oder Pyridyl substituierte Alkylgruppe mit bis zu 6 Kohlenstoffatomen darstellt oder $R_2$ für eine Phenyl-, Naphthyl-, Cyclohexyl- oder Pyridylgruppe steht;

$R_3$ ein nicht substituiertes oder durch eine freie oder eine Schutzgruppe tragende Aminogruppe, durch ein freies Carboxyl, durch eine Hydroxygruppe, durch Alkylthio mit bis zu 6 Kohlenstoffatomen, durch Phenyl, durch Naphthyl oder durch 4-Imidazolyl substituiertes Alkyl mit bis zu 6 Kohlenstoffatomen darstellt;

$R_4$ Hydroxyl, Alkoxy mit bis zu 6 Kohlenstoffatomen, Benzyloxy oder eine freie oder durch 1 oder 2 Alkyle mit bis zu 6 Kohlenstoffatomen substituierte Aminogruppe darstellt;

$Z_1$ für Isopropyl, Phenyl oder Cyclohexyl steht, welches jeweils mit der Gruppe

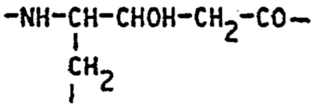

den Rest der Aminosäure Statin, nämlich (3S,4S)-4-Amino-3-hydroxy-6-methylheptansäure, oder der (3S,4S)-4-Amino-3-hydroxy-5-phenylheptansäure (AHPPA) oder der (3S,4S)-4-Amino-5-cyclohexyl-3-hydroxy-pentansäure (ACHPA) bildet;

X–Y ein Dipeptid, ausgewählt aus Ala–Sta, Ala–Leu, Leu–Phe, Val–Sta, Abu–Sta, Ile–Ser, Phg–Sta, ist;

oder eines der möglichen pharmazeutisch akzeptablen Salze des Peptidderivats mit mineralischen oder organischen Säuren oder mit Alkali- oder Erdalkalimetallen.

2. Peptidderivat nach Anspruch 1, dadurch gekennzeichnet, daß $R_1$ ausgewählt ist aus den folgenden Gruppen:

– 4-(2-Pyridyl)-4-oxo-butyryl,
– 4-(2-Pyridyl)-4-hydroxy-butyryl,
– 3-(3-Pyridyl)-propionyl,
– 4-(3-Pyridyl)-butyryl,
– Nikotinoyl.

3. Peptidderivat, dadurch gekennzeichnet, daß es N-(Acetyl-(N-Boc-piperidin-3-yl))-Phe-Nle-Sta-Leu-Phe-OMe ist.

4. Verfahren zur Herstellung eines Peptidderivats nach Anspruch 1, dadurch gekennzeichnet, daß man, ausgehend von einer Verbindung der Formel H–Y–R'$_4$, worin R'$_4$ Alkoxy mit bis zu 6 Kohlenstoffatomen, Benzyloxy oder eine freie oder durch 1 oder 2 Alkyle mit bis zu 6 Kohlenstoffatomen substituierte Aminogruppe darstellt und Y ausgewählt ist aus den Resten der Aminosäuren Sta, Leu, Phe und Ser, Schritt für Schritt die verschiedenen Aminosäuren oder Fragmente der Sequenz des Peptids, entsprechend geschützt, aneinanderkoppelt, wobei das bei jedem Schritt erhaltene Produkt mittels bekannter Verfahren entschützt wird, bevor es einer neuerlichen Kopplung unterzogen wird, wobei jeder Kopplungsvorgang unter Verwendung entweder eines aktivierten Esters der zu koppelnden Aminosäure oder der N-geschützten Aminosäure in Gegenwart von Dicyclohexylcarbodiimid durchgeführt wird, daß man gegebenenfalls die erhaltene Verbindung verseift, um das Peptid der Formel I zu erhalten, in dem $R_4$ = OH, und/oder daß man gegebenenfalls das erhaltene Peptid mit mineralischen oder organischen Säuren oder Alkali- oder Erdalkalimetallen in eines seiner pharmazeutisch akzeptablen Salze überführt.

5. Verfahren zur Herstellung des Peptidderivats der Formel N-(Acetyl-(N-Boc-piperidin-3-yl))-Phe-Nle-Sta-Leu-Phe-OMe, dadurch gekennzeichnet, daß man, ausgehend von einer Verbindung der Formel H–Y–R'$_4$, in der R'$_4$ Methoxy darstellt und Y Phe ist, Schritt für Schritt die verschiedenen Aminosäuren oder Fragmente der Sequenz des Peptids, entsprechend geschützt, aneinanderkoppelt, wobei das bei jedem Schritt erhaltene Produkt mittels bekannter Verfahren entschützt wird, bevor es einer neuen Kopplung unterzogen wird, wobei jeder Kopplungsvorgang unter Verwendung entweder eines aktivierten Esters der zu koppelnden Aminosäure oder der N-geschützten Aminosäure in Gegenwart von Dicyclohexylcarbodiimid durchgeführt wird.

6. Pharmazeutische Zusammensetzungen, dadurch gekennzeichnet, daß sie ein Produkt nach Anspruch 1 als aktives Ingrediens enthalten.

7. Pharmazeutische Zusammensetzungen nach Anspruch 6, die insbesondere zur Behandlung des arteriellen Drucks verwendbar sind, dadurch gekennzeichnet, daß sie 1 bis 1000 mg Produkt nach Anspruch 1 pro Dosiseinheit in Mischung mit einem pharmazeutischen Exzipienten enthalten.

**Patentansprüche für den Vertragsstaat: AT**

1. Verfahren zur Herstellung von Peptidderivaten der Formel

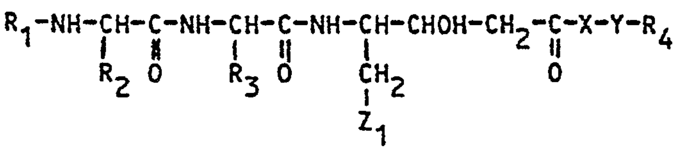

(I)

worin:

$R_1$ eine der folgenden Gruppen darstellt:

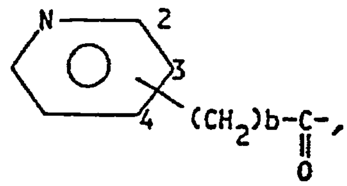

worin b = 0, 1, 2, 3, 4, 5 oder 6

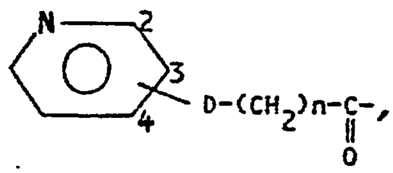

worin D = 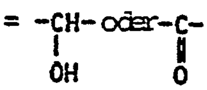

und n = 1, 2, 3, 4 oder 5

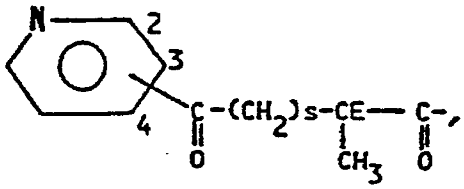

worin E = –H oder $-CH_3$
und s = 1, 2, 3 oder 4

$R_2$ eine nicht substituierte oder durch Phenyl, Naphthyl, Cyclohexyl oder Pyridyl substituierte Alkylgruppe mit bis zu 6 Kohlenstoffatomen darstellt oder $R_2$ für eine Phenyl-, Naphthyl-, Cyclohexyl- oder Pyridylgruppe steht;

$R_3$ ein nicht substituiertes oder durch eine freie oder eine Schutzgruppe tragende Aminogruppe, durch ein freies Carboxyl, durch eine Hydroxygruppe, durch Alkylthio mit bis zu 6 Kohlenstoffatomen, durch Phenyl, durch Naphthyl oder durch 4-Imidazolyl substituiertes Alkyl mit bis zu 6 Kohlenstoffatomen darstellt;

$R_4$ Hydroxyl, Alkoxy mit bis zu 6 Kohlenstoffatomen, Benzyloxy oder eine freie oder durch 1 oder 2 Alkyle mit bis zu 6 Kohlenstoffatomen substituierte Aminogruppe darstellt;

$Z_1$ für Isopropyl, Phenyl oder Cyclohexyl steht, welches jeweils mit der Gruppe

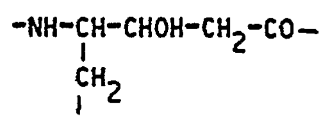

den Rest der Aminosäure Statin, nämlich (3S,4S)-4-Amino-3-hydroxy-6-methylheptansäure, oder der (3S,4S)-4-Amino-3-hydroxy-5-phenylpentansäure (AHPPA) oder der (3S,4S)-4-Amino-5-cyclohexyl-3-hydroxy-pentansäure (ACHPA) bildet;

X–Y ein Dipeptid, ausgewählt aus Ala–Sta, Ala–Leu, Leu–Phe, Val–Sta, Abu–Sta, Ile–Ser, Phg–Sta, ist;

oder eines der möglichen pharmazeutisch akzeptablen Salze des Peptidderivats mit mineralischen oder organischen Säuren oder mit Alkali- oder Erdalkalimetallen, dadurch gekennzeichnet, daß man, ausgehend von einer Verbindung der Formel H–Y–R'$_4$, worin R'$_4$ Alkoxy mit bis zu 6 Kohlenstoffatome, Benzyloxy oder eine freie oder durch 1 oder 2 Alkyle mit bis zu 6 Kohlenstoffatomen substituierte Aminogruppe darstellt und Y ausgewählt ist aus den Resten der Aminosäuren Sta, Leu, Phe und Ser, Schritt für Schritt die verschiedenen Aminosäuren oder Fragmente der Sequenz des Peptids, entsprechend geschützt, aneinanderkoppelt, wobei das bei jedem Schritt erhaltene Produkt mittels bekannter Verfahren entschützt wird, bevor es einer neuerlichen Kopplung unterzogen wird, wobei jeder Kopplungsvorgang unter Verwendung entweder eines aktivierten Esters der zu koppelnden Aminosäure oder der N-geschützten Aminosäure in Gegenwart von Dicyclohexylcarbodiimid durchgeführt wird, daß man gegebenenfalls die erhaltene Verbindung verseift, um das Peptid der Formel I zu erhalten, in dem $R_4$ = OH, und/oder daß man gegebenenfalls das erhaltene Peptid mit mineralischen oder organischen Säuren oder Alkali- oder Erdalkalimetallen in eines seiner pharmazeutisch akzeptablen Salze überführt.

2. Verfahren nach Anspruch 1, dadurch gekennzeichnet, daß die letzte zu koppelnde Aminosäure derart gewählt wird, daß $R_1$ eine der folgenden Gruppen darstellt:

– 4-(2-Pyridyl)-4-oxo-butyryl,

– 4-(2-Pyridyl)-4-hydroxy-butyryl,

– 3-(3-Pyridyl)-priopionyl,
– 4-(3-Pyridyl)-butyryl,
– Nikotinoyl.

3. Verfahren zur Herstellung des Peptidderivats der Formel N-(Acetyl-(N-Boc-piperidin-3-yl))-Phe-Nle-Sta-Leu-Phe-OMe, dadurch gekennzeichnet, daß man, ausgehend von einer Verbindung der Formel H–Y–$R'_4$, in der $R'_4$ Methoxy darstellt und Y Phe ist, Schritt für Schritt die verschiedenen Aminosäuren oder Fragmente der Sequenz des Peptids, entsprechend geschützt, aneinanderkoppelt, wobei das bei jedem Schritt erhaltene Produkt mittels bekannter Verfahren entschützt wird, bevor es einer neuen Kopplung unterzogen wird, wobei jeder Kopplungsvorgang unter Verwendung entweder eines aktivierten Esters der zu koppelnden Aminosäure oder der N-geschützten Aminosäure in Gegenwart von Dicyclohexylcarbodiimid durchgeführt wird.

4. Verwendung der mit dem Verfahren nach einem der Ansprüche 1 bis 3 hergestellten Peptide zur Herstellung von pharmazeutischen Zusammensetzungen für die Behandlung des arteriellen Drucks.